(19) **Europäisches Patentamt / European Patent Office / Office européen des brevets**

(11) **EP 3 305 300 B1**

(12) # EUROPEAN PATENT SPECIFICATION

(45) Date of publication and mention
of the grant of the patent:
**04.10.2023 Bulletin 2023/40**

(21) Application number: **16799403.7**

(22) Date of filing: **26.05.2016**

(51) International Patent Classification (IPC):
**A61K 31/616** (2006.01)  **A61P 27/02** (2006.01)

(52) Cooperative Patent Classification (CPC):
**A61K 31/616; A61P 27/02**

(86) International application number:
**PCT/ES2016/070396**

(87) International publication number:
**WO 2016/189181 (01.12.2016 Gazette 2016/48)**

(54) **INTRAVITREAL LYSINE ACETYLSALICYLATE FOR USE IN THE TREATMENT OF DIABETIC RETINOPATHY**

INTRAVITREALES LYSINACETYLSALICYLAT ZUR VERWENDUNG BEHANDLUNG VON DIABETISCHER RETINOPATHIE

ACÉTYLSALICYLATE DE LYSINE À ADMINISTRATION INTRAVITRÉENNE POUR UTILISATION DANS UN MÉTHODE DE TRAITEMENT DE LA RÉTINOPATHIE DIABÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.05.2015 ES 201530727**

(43) Date of publication of application:
**11.04.2018 Bulletin 2018/15**

(73) Proprietor: **Fundación Para El Fomento De La Investigación Sanitaria Y Biomédica De La Comunitat Valenciana
46010 Valencia (ES)**

(72) Inventors:
• **MARTÍNEZ TOLDOS, José Juan**
46010 Valencia (ES)
• **FERNANDEZ MARTÍNEZ, Cristian**
46010 Valencia (ES)
• **RUIZ MORENO, José María**
02071 Albacete (ES)

(74) Representative: **Pons Ariño, Angel
Pons Patentes y Marcas Internacional, S.L.
Glorieta Rubén Dario 4
28010 Madrid (ES)**

(56) References cited:
JP-A- S61 260 020

• ANANT PAI ET AL: "Current concepts in intravitreal drug therapy for diabetic retinopathy", SAUDI JOURNAL OF OPHTHALMOLOGY, ELSEVIER, AMSTERDAM, NL, vol. 24, no. 4, 1 October 2010 (2010-10-01), pages 143-149, XP002674122, ISSN: 1319-4534, DOI: 10.1016/J.SJOPT.2010.06.003 [retrieved on 2010-06-30]
• Bfarm: "Core Safety Profile of lysine acetylsalicylate", , 2 October 2013 (2013-10-02), XP055516987, Retrieved from the Internet: URL:http://www.bfarm.de/SharedDocs/Downloads/EN/Drugs/vigilance/PSURs/csp/k-l/lysine-acetylsalicylate.pdf?__blob=publicationFile&v=3 [retrieved on 2018-10-18]
• KRALINGER MARTINA T ET AL.: 'Intravitreal acetylsalicylic acid in silicone oil: Pharmacokinetics and evaluation of its safety by ERG and histology.' GRAEFE'S ARCHIVE FOR CLINICAL AND EXPERIMENTAL OPHTHALMOLOGY vol. 239, no. 3, March 2001, ISSN 0721-832X pages 208 - 216, XP055331711

**(Cont. next page)**

- **KRALINGER MARTINA THERESA ET AL.: 'Safety and feasibility of a novel intravitreal tamponade using a silicone oil/acetyl-salicylic acid suspension for proliferative vitreoretinopathy: first results of the Austrian Clinical Multicenter Study.' GRAEFE'S ARCHIVE FOR CLINICAL AND EXPERIMENTAL OPHTHALMOLOGY vol. 248, no. 8, August 2010, ISSN 0721-832X pages 1193 - 1198, XP019848027**

**Description**

The present invention relates to a composition comprising lysine acetylsalicylate for use in a form suitable for intravitreal administration for the treatment of diabetic retinopathy.

**STATE OF THE ART**

Diabetic retinopathy is the leading cause of legal blindness in individuals younger than 50 years of age in developed countries and one of the leading causes of blindness in the world. Diabetes mellitus affects between 6 and 18% of the population in Spain, wherein a third of Spain's population have diabetic retinopathy and another third, in turn, have diabetic macular edema, which is the main cause of vision loss in diabetic individuals. The WHO estimates that there are 346 million of diabetics in the world.

At the onset of type 1 diabetes, only between 0 and 3% of those affected exhibit some degree of diabetic retinopathy, but 67.1% of these patients suffer from diabetic retinopathy before their diabetes has progressed for 5 years. In the case of type 2 diabetes, between 6 and 30% of the patients already exhibit retinopathy at the time of diagnosis and in the cases of more than 20 years of progression of type 2 diabetes, the prevalence of diabetic retinopathy is 82%.

To date, there are three medicaments on the market which are used for the treatment of diabetic macular edema (DME), in some patients with diabetic retinopathy. One of these medicaments is ranibizumab (Lucentis®), a fragment of humanized immunoglobulin whose fundamental action is to block all subtypes of the vascular endothelial growth factor (VEGF). The lesion of the small blood vessels (microangiopathy) that occurs with the progress of diabetic retinopathy restricts blood flow to the damaged tissue, causing ischemia. These ischemic tissues express angiogenic molecules in order to form new vessels which provide nutrients and solve the ischemia. These new vessels are immature in nature and far from providing suitable blood flows which allow the survival of the tissue, they exhibit a high vascular permeability which contributes to the edema formed in the macula. Ranibizumab blocks the angiogenesis induced by VEGF-A by producing a regression of the neovessels and thereby inducing improvements to the retinal edema. However, the progressive course of the disease is not modified as far as it does not resolve the ischemia nor does it change other circumstances and molecular occurrences which it is known that occur in diabetic retinopathy. Another medicament is aflibercept (Eylea®) which acts similarly to ranibizumab, but in turn inhibits another molecule in addition to VEGF-A, such as PIG-F (placental growth factor); this medicament exhibits comparable effects to ranibizumab and also has the same limitations. Another indicated therapeutic option is a steroid, dexamethasone, which is administered in intravitreal injection in a sustained release device (Ozurdex®), which is approved for its use as a first line medicament or in the case the previous anti-VEGF fails. Another sustained release system of another steroid is being marketed, in this case fluocinolone (Lluvien®). A drawback of both steroids is the high rate of side effects they induce, such as an increase of intraocular pressure and cataracts. Bevacizumab (Avastin®), which is an inhibitor of all the isoforms of VEGF, has also been used off-label; although there is evidence of non-inferiority with respect to ranibizumab, there is no official approval for its intraocular use.

There are scientific evidences that substantiate the relevance of inflammatory cellular phenomena in the pathogeny of diabetic retinopathy; therefore products with anti-inflammatory action such as steroids or the invention in question acquire special use and relevance especially for their efficacy in more advanced phases of the disease where the inflammatory phenomena are highly significant.

There are increasingly more histochemical studies which demonstrate that there is a basic inflammatory problem which acts as the main trigger factor of diabetic retinopathy, attracting the specialized cells in the inflammatory response, the leucocytes, and over time leading to a failure and loss of vascular endothelium, an increase of vascular permeability (causing edema) and an increase of platelet aggregation (contributing to the obliteration of the vessels and to retinal ischemia).

In addition, unlike ranibizumab, acetylsalicylate is capable of acting on various therapeutic targets involved in the inflammation, targets which are present even in very early stages of this disease. Therefore, acetylsalicylate is present as a potentially useful medicament in initial and advanced stages of diabetic retinopathy.

The oral use of acetylsalicylic acid has been described in the context of diabetic retinopathy both with a preventative and a therapeutic profile (see L Zheng, SJ Howell, DA Hatala, K Huang, T S Kern. Salicylate-based anti-inflammatory medicaments inhibit the early lesion of diabetic retinopathy. Diabetes 2007; 56:337-335. W Sun, C Gerhardinger, Z Dagher, T Hoehn, M Lorenzi. Aspirin at low-intermediate concentrations protects retinal vessels in experimental diabetic retinopathy through non-platelet-mediated effects. Diabetes 2005; 54:3418-3426. TS Kern, RL Engerman. Pharmacological inhibition of diabetic retinopathy, aminoguanidine and aspirin. Diabetes 2001; 50:1636-1642.). It has thus been demonstrated that salicylate has a beneficial effect on the progressive course of the disease and no counterproductive effect on it. However, it is based on the oral use of the salicylate in humans and in animals, which in many cases involves high and non-tolerable doses with frequent and potentially serious adverse effects.

A composition of acetylsalicylic acid (AS) and silicone oil (SiO) for intravitreal use has been described (see, for example, Kralinger M. T et al Graefe's Arch Clin Exp Ophthalmol 2001;239:208-216). In cases of very advanced diabetic retinopathy which causes very serious complications in the retina, such as tractional detachment, breakages and fibrovascular proliferation, surgical intervention is necessary. This intervention involves the elimination of the vitreous humor in the patient (vitrectomy), the removal of anomalous fibrovascular tissue present in the retina of these patients and the placement of a substitute of the vitreous humor. The silicone oil is used to substitute the vitreous in these cases. The SiO is characterized in that it is a dense, inert and stable element which helps to maintain the correctly applied retina. It is an immiscible medium for all types of substances, whether they are medicaments or inflammatory and angiogenic molecules, which prevents them from spreading through the vitreous cavity. It does not have any chemical action on the retina, it is not capable of acting as a reserve for medicaments and it is not useful as a solvent (Abrams GW, Azen SP, McCuen BW, Flynn H, Lai MY, Ryan SJ, Silicone Study Group: Vitrectomy with silicone oil or long-acting gas in eyes with severe proliferative vitreoretinopathy: Results of additional long-term follow-up (Silicone Study Report #11). Archives of Ophthalmology 115: 335-344, 1997.). Further, Anant Pai *et al.* (see Anant Pai et al. "Current concepts in intravitreal drug therapy for diabetic retinopathy", Saudi Journal of Ophthalmology, 2010, 24(4), pages 143-149) discloses the intravitreal administration of active agents, such as steroids, antiVEGFs and enzymes for use in the treatment of diabetic retinopathy.

It would therefore be desirable to have a composition for intravitreal administration of acetylsalicylate for use in the treatment of diabetic retinopathy which can be injected in the presence of the vitreous humor, that is, which can be administered in less advanced stages of the disease with the purpose of avoiding the progression of the disease and preventing patients from needing surgery.

## DESCRIPTION OF THE INVENTION

In a first aspect, the present invention relates to a composition comprising lysine acetylsalicylate for use in the treatment of a disease of the retina, wherein the composition is administered intravitreally and the disease of the retina is diabetic retinopathy.

In another embodiment, the invention relates to the composition for the use as previously defined for the treatment of a disease selected from uveitic macular edema, non-infectious retinitis, non-infectious retinocoroiditis, proliferative vitreoretinopathy, chronic Irving Gass syndrome, chronic central serous chorioretinopathy and diabetic retinopathy; and preferably for the treatment of diabetic retinopathy.

In another embodiment, the invention relates to the composition for the use as previously defined, wherein the concentration of lysine acetylsalicylate is between 12 mg/ml of composition and 24 mg/ml of composition in the vitreous humor.

In another embodiment, the invention relates to the composition for the use as previously defined, wherein the concentration of lysine acetylsalicylate is 12 mg/ml of composition, 18 mg/ml of composition or 24 mg/ml of composition.

In another embodiment, the invention relates to the composition for the use as previously defined, wherein the pH of the composition is between 6 and 6.2; and preferably wherein the pH of the composition is 6.

In another embodiment, the invention relates to the composition for the use as previously defined, wherein:

the concentration of lysine acetylsalicylate is between 12 mg/ml of composition and 24 mg/ml of composition, and preferably wherein the concentration of lysine acetylsalicylate is 12 mg/ml of composition, 18 mg/ml of composition or 24 mg/ml of composition; and
the pH of the composition is between 6 and 6.2, and preferably wherein the pH of the composition is 6.

In another embodiment, the invention relates to the composition for the use as previously defined, which further comprises physiological saline solution and preferably which further comprises between 2.6 ml and 5.6 ml of physiological saline solution.

In another embodiment, the invention relates to the composition for the use as previously defined, which further comprises water and preferably which further comprises 5 ml of water.

In another embodiment, the invention relates to the composition for the use as previously defined wherein:

the concentration of lysine acetylsalicylate is between 12 mg/ml of composition and 24 mg/ml of composition, and preferably wherein the concentration of lysine acetylsalicylate is 12 mg/ml of composition, 18 mg/ml of composition or 24 mg/ml of composition; and
which further comprises physiological saline solution and preferably which fuerther comprises 2.6 ml, 4.5 ml or 5.6 ml of physiological saline solution.

In another embodiment, the invention relates to the composition for the use as previously defined wherein:

the concentration of lysine acetylsalicylate is between 12 mg/ml of composition and 24 mg/ml of composition, and preferably wherein the concentration of lysine acetylsalicylate is 12 mg/ml of composition, 18 mg/ml of composition or 24 mg/ml of composition;

the pH of the composition is between 6 and 6.2, and preferably wherein the pH of the composition is 6; and wherein further comprises physiological saline solution and preferably further comprises 2.6 ml, 4.5 ml or 5.6 ml of physiological saline solution.

In another embodiment, the invention relates to the composition for the use as previously defined wherein:

the concentration of lysine acetylsalicylate is between 12 mg/ml of composition and 24 mg/ml of composition, and preferably wherein the concentration of lysine acetylsalicylate is 12 mg/ml of composition, 18 mg/ml of composition or 24 mg/ml of composition; and
wherein further comprises water and preferably which also comprises 5 ml of water.

In another embodiment, the invention relates to the composition for the use as previously defined wherein:

the concentration of lysine acetylsalicylate is between 12 mg/ml of composition and 24 mg/ml of composition, and preferably wherein the concentration of lysine acetylsalicylate is 12 mg/ml of composition, 18 mg/ml of composition or 24 mg/ml of composition;
the pH of the composition is between 6 and 6.2, and preferably wherein the pH of the composition is 6; and wherein further comprises water and preferably which also comprises 5 ml of water.

In another embodiment, the invention relates to the composition for the use as previously defined wherein:

the concentration of lysine acetylsalicylate is between 12 mg/ml of composition and 24 mg/ml of composition, and preferably wherein the concentration of lysine acetylsalicylate is 12 mg/ml of composition, 18 mg/ml of composition or 24 mg/ml of composition;
wherein further comprises physiological saline solution and preferably further comprises 2.6 ml, 4.5 ml or 5.6 ml of physiological saline solution; and wherein further comprises water and preferably further comprises 5 ml of water.

In another embodiment, the invention relates the composition for the use as previously defined wherein:

the concentration of lysine acetylsalicylate is between 12 mg/ml of composition and 24 mg/ml of composition, and preferably wherein the concentration of lysine acetylsalicylate is 12 mg/ml of composition, 18 mg/ml of composition or 24 mg/ml of composition;
the pH of the composition is between 6 and 6.2, and preferably wherein the pH of the composition is 6,
wherein further comprises physiological saline solution and preferably further comprises 2.6 ml, 4.5 ml or 5.6 ml of physiological saline solution; and wherein further comprises water and preferably further comprises 5 ml of water.

In another embodiment, the invention relates to the composition for the use as previously defined wherein:

the concentration of lysine acetylsalicylate is between 12 mg/ml of composition and 24 mg/ml of composition, and preferably wherein the concentration of lysine acetylsalicylate is 12 mg/ml of composition, 18 mg/ml of composition or 24 mg/ml of composition;
the pH of the composition is between 6 and 6.2, and preferably wherein the pH of the composition is 6;
wherein further comprises physiological saline solution and preferably further comprises 2.6 ml, 4.5 ml or 5.6 ml of physiological saline solution; and
wherein further comprises water and preferably further comprises 5 ml of water, for the manufacture of a medicament for the treatment of a disease of the retina, preferably for the treatment of a disease selected from uveitic macular edema, non-infectious retinitis, non-infectious retinocoroiditis, proliferative vitreoretinopathy, chronic Irving Gass syndrome, chronic central serous retinopathy and diabetic retinopathy; and more preferably for the treatment of diabetic retinopathy.

In another embodiment, the invention relates to the composition for the use as previously defined, wherein the total amount of acetylsalicylic acid injected into the vitreous humor is between 0.6 mg and 1.2 mg.
In another embodiment, the invention relates to the composition for the use as previously defined, wherein the total amount of acetylsalicylic acid injected into the vitreous humor is 0.6 mg, 0.9 mg or 1.2 mg.
In another embodiment, the invention relates to the composition for the use as previously defined, wherein:

the total amount of acetylsalicylic acid injected into the vitreous humor is between 0.6 mg and 1.2 mg; and the final volume of the composition is at least 0.05 ml.

In another embodiment, the invention relates to the composition for the use as previously defined, wherein the total amount of acetylsalicylic acid injected into the vitreous humor is 0.6 mg, 0.9 mg or 1.2 mg; and the final volume of the composition is at least 0.05 ml.

The use of lysine as an ingredient of the composition facilitates solubility and improves the stability of the active ingredient forming the salt of the lysine acetylsalicylate.

The composition can be used in patients who have not been necessary to remove the vitreous humor. The vitreous humor acts as a reserve of medicaments such that it facilitates the persistence and spreading of the lysine acetylsalicylate of the invention inside the eye.

As previously mentioned, the composition of the invention is administered in an intravitreal parenteral route. Thus, the injectable preparations for parenteral administration, in accordance with the present invention, comprise sterile solutions, suspensions or emulsions in an aqueous or non-aqueous solvent such as propylene glycol or polyethylene glycol or vegetable oils. These compositions can also contain coadjuvants such as humectants, emulsifiers, dispersants and preservatives. They could be sterilized by any of the methods known or prepared as sterile solid compositions which will be dissolved in water or any other injectable medium that is sterile immediately prior to use. It is also possible to use sterile raw materials and keep them in these conditions for the whole manufacturing process.

The composition mentioned can be prepared following the method which is detailed below:

Pharmaceutical form: sterile solution.

Formula:

| Lysine acetylsalicylate | 900 mg |
| Water for injection | 5 ml |
| Physiological saline solution | 6 ml |

Material: 1.5 ml syringes, needles, 0.22 micron filter, sterile bottle.

Preparation: laminar flow hood,

1. Load 5 ml of sterile water for injection and inject it into the vial which contains 900 ml of lysine acetylsalicylate. Stir gently until completely dissolved, thus obtaining a concentration of acetylsalicylic acid of 100 mg/ml of composition = lysine acetylsalicylate of 180 mg/ml of composition

2. For a solution of 12 mg/ml: load 0.4 ml (72 mg of lysine acetylsalicylate) of the solution obtained and introduce it into a sterile vial, add 5.6 ml of physiological saline solution with a resulting pH of 6.

3. For a solution of 24 mg/ml: load 0.4 ml (72 mg of lysine acetylsalicylate) of the solution obtained and introduce it into a sterile vial, add 2.6 ml of physiological saline solution with a resulting pH of 6.

4. For a more preferred solution of 18 mg/ml: load 0.5 ml (90 mg of lysine acetylsalicylate) of the solution obtained and introduce it into a sterile vial, add 4.5 ml of physiological saline solution with a resulting pH of 6.

5. The final preparation will be filtered (0.22 micron filter) prior to packaging it in the 1 ml syringe which will be loaded with a volume of 0.05 ml and covered with a sterile cover.

6. Package and label. The label will indicate the route of administration, the active ingredient, the final concentration, the preparation date, the type of storage and the expiry.

Packaging: Syringe of suitable volume.

Throughout the description and the claims, the word "comprises" and its variants do not intend to exclude other technical characteristics, additions, components or steps. For a person skilled in the art, other objects, advantages and characteristics of the invention will emerge in part from the description and in part from the practice of the invention. The following examples and figures are provided by way of illustration and do not intend to limit the present invention.

**BRIEF DESCRIPTION OF THE FIGURES**

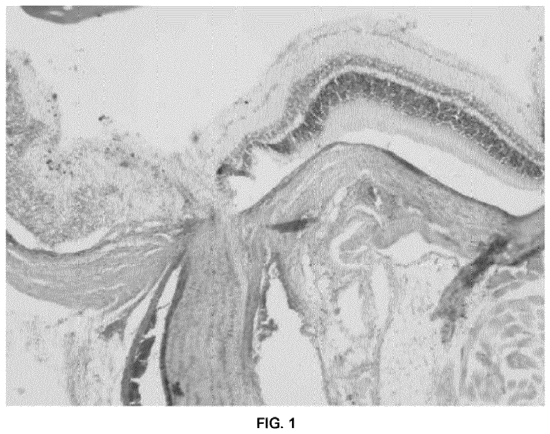

**FIG. 1** shows the histological cut stained with H&E of the central retina (see the optical nerve in the central part of the image).

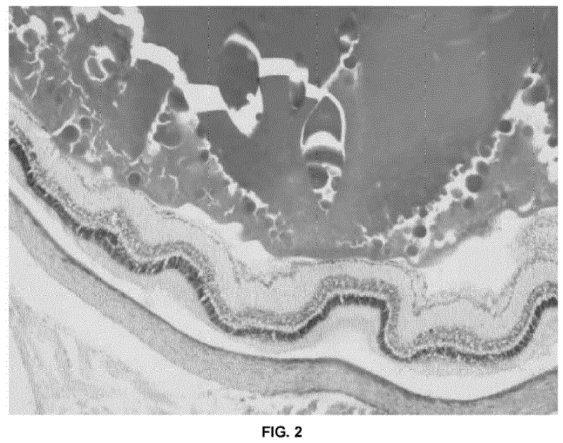

**FIG. 2** shows the histological cut stained with H&E of the peripheral retina.

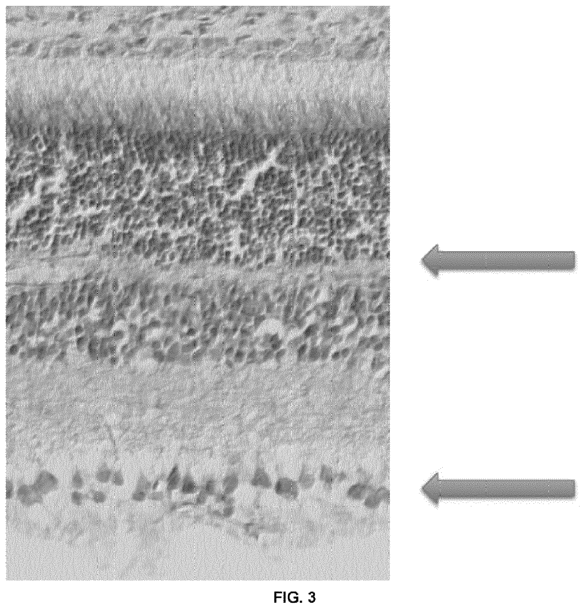

**FIG. 3** shows the histological cut stained with H&E where the ganglion cell layer (lower arrow) and external plexiform layer (upper arrow) are indicated.

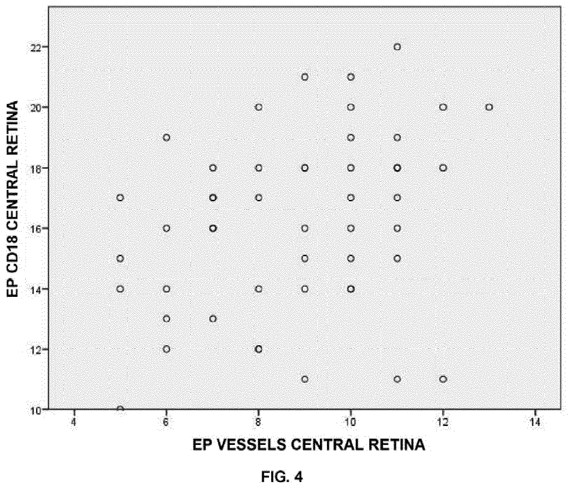

**FIG. 4** shows the correlation and the result of the Spearman test of the external plexiform central retina: n=50; r=0.341; p=0.015. This means that the correlation between both is low (the determination coefficient which would be r2 is 0.116, that is, 11.6% of the variability of the data which is explained by the relation between both variables).

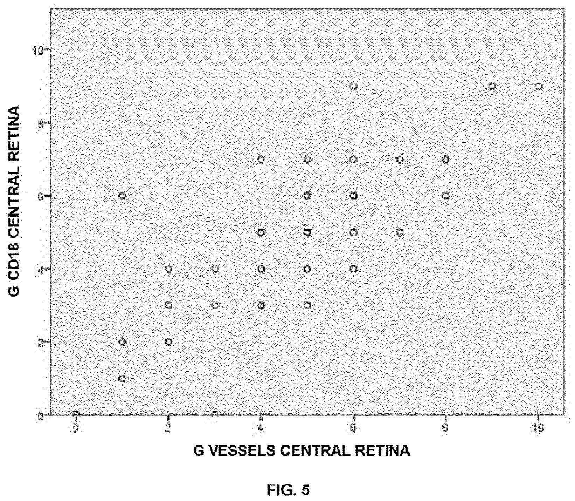

**FIG. 5** shows the correlation and the result of the Spearman test of the ganglion central retina: n=50; r=0.803; p<0.001. This means that the correlation between both is high (the determination coefficient which would be r2 is 0.645, that is, 64.5% of the variability of the data which is explained by the relation between both variables).

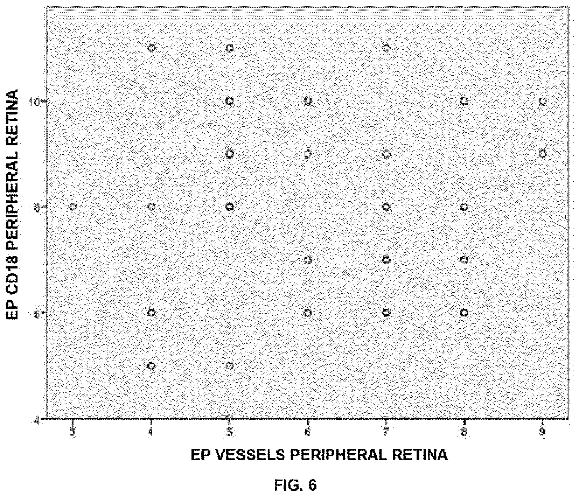

**FIG. 6** shows the correlation and the result of the Spearman test of the external plexiform peripheral retina: n=50; r=0.005; p=0.973. This means that the correlation between both is low, almost zero (the determination coefficient which would be r2 is 0.000025, that is, 0.0025% of the variability of the data which is explained by the relation between both variables).

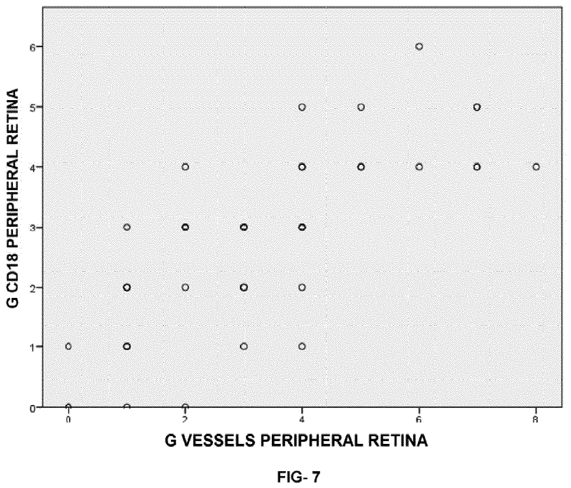

**FIG. 7** shows the correlation and the result of the Spearman test of the ganglion peripheral retina: n=50; r=0.737; p=0.001. What this means is that the correlation between both is not very high (the determination coefficient which would be r2 is 0.543, that is, 54.3% of the variability of the data which is explained by the relation between both variables).

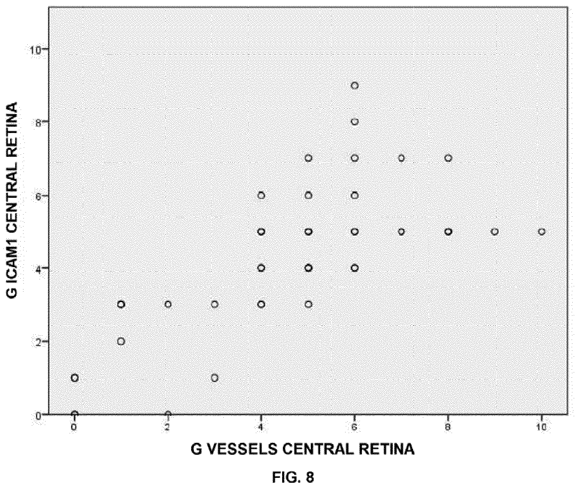

**FIG. 8** shows the relation between ICAM1 and vessels of the ganglion central retina: n=50; r=0.616; p<0.001. This means that the correlation between both is not very high (the determination coefficient which would be r2 is 0.379, that is to say, 37.9% of the variability of the data which is explained by the relation between both variables).

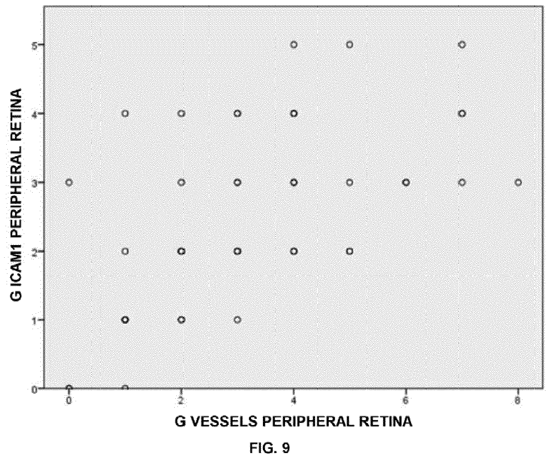

**FIG. 9** shows the relation between ICAM1 and vessels of the ganglion peripheral retina: n=50; r=0.341; p<0.015. What this means is that the correlation between both is low (the determination coefficient which would be r2 is 0.116, that is, 11.6% of the variability of the data which is explained by the relation between both variables).

## EXAMPLES

The invention will be illustrated below by means of tests carried out by the inventors which reveal the effectiveness of the product of the invention.

EXAMPLE 1

*Objectives*

Assess the effects of the intravitreal injection of a solution of lysine acetylsalicylate in an experimental model of diabetic retinopathy on 3 histological variables in the retina of the treated animals, comparing them with a control group. The selected variables, which have demonstrated in various studies to be primordially affected in the diabetic retina, are:

1. CD18 leucocyte marker: the leucocyte is the main effector cell of the inflammatory response of the tissues, something which also occurs in the retina. CD18 is a surface molecule which is expressed in the membranes of the leucocytes activated in tissues where there is inflammation, as is the case with the diabetic retina. Determine absolute and relative number helps to know whether the injected solution is capable of reducing the presence of leucocytes activated in determined layers of the retina as part of their anti-inflammatory action.

2. ICAM-1 endothelial marker: it has been demonstrated that the endothelial cells which cover the inner of the blood vessels are affected in diabetic retinopathy and for this reason they are very relevant in the pathogeny of the disease. ICAM-1 is a transmembrane protein present in the endothelial cells and practically specific to them. The immunohistochemical staining allows the endothelial cells present in the different layers of examined tissues to be quantitatively indicated and assessed and it gives us information concerning the vascularization of the examined tissues and the integrity of the vascular wall.

3. Vascular density: the chronic inflammation caused in diabetic retinopathy involves obliteration and death of the blood vessels. This leaves more or less extensive areas of tissue that do not receive any type of blood supply. This scenario is a final stage of the phenomenon known as diabetic microangiopathy. Thus, determining the number of vessels present in each section of tissue can give us information concerning whether or not the medicament is capable of positively or negatively influencing the capillary density of the retina (vessels/portion of tissue).

*Methodology*

1. Experimental model

Pursuant to approval of the Bioethics Committee for Research of the University of Miguel Hernández de Elche and based on the international standards for the use of animal experimentation, 14 confined male Wistar rat specimens weighing 200 grams were induced to suffer diabetes by means of injecting intraperitoneal streptozotocin (75 mg/kg) in a single dose. The animals were monitored during the whole study continuously in terms of weight, blood sugar, loss of hair and formation of cataracts. All the animals were exposed to the same parameters of light, darkness and availability of water and food.

2. Intravitreal injection technique

For the intravitreal injection, the animals were anesthetized with a mixture in a proportion of 1:1 of xylazine hydrochloride (4 mg/kg) and ketamine hydrochloride (10 mg/kg). Under mydriasis with 0.5% tropicamide, the intravitreal injection of the lysine acetylsalicylate solution was carried out under a surgical microscope with a micro injection syringe (Hamilton Co., Reno, NV) and a 32 gauge needle at 1 millimeter from the temporal limbus. Only half the diabetic animals were treated with the intravitreal solution, the rest of the animals were left to develop diabetic retinopathy naturally. The process of intravitreal injection was repeated in the same way, technique and dose in the same animals 4 weeks after the initial treatment. Summary of the organization of the experimental test:

- Initially, the animals were evaluated prior to including them in the experimental test to discard those with defects or basic ocular changes.
- The inducement of diabetes was carried out in all the animals by means of injecting intraperitoneal streptozotocin.
- The blood sugar indices were compared at 24 and 48 hours of the injection to assess whether the inducement had been successful.
- Once diabetes was confirmed, the animals were divided into two groups formed by 8 rats each.
- Following the diabetic retinopathy model in rats, diabetes was left to develop naturally in both groups over the first 12 weeks. It is in this period that it has been demonstrated that they start to show the signs of diabetic retinopathy.
- At this point, an intravitreal injection of acetylsalicylate in a solution with lysine was given to one of the groups in both eyes of each animal.
- At 4 weeks, (week 16 of the induction) the intravitreal injection of salicylate was repeated in the same group of animals.
- At 4 weeks from the last injection (20 weeks following induction), the animals of both groups were sacrificed.

3. Calculation of dose and preparation of the solution

There are no dosimetric references or studies carried out hitherto for making this calculation. However, we can perform the following approximation:
Therapeutic range:

Analgesic and antipyretic action 2.5-5.0 mg/dl
Anti-inflammatory action 15-30 mg/dl
Toxic range
40-50 mg/dl

With a concentration of 25 mg/dl (0.25 $\mu$g/$\mu$l) in the biophase, we could cover a therapeutic range suitable for assessing the existence of significant effects in the variables under examination.
The volume of vitreous humor existing in the eye of the adult rat is 56.5 + 2 microliters (Oxygen Distribution in the Mouse Retina. Dao-Yi Yu and Stephen J. Cringle. Invest Ophthalmol Vis Sci. 2006; vol. 47; 1109-1112)

If a monocompartimental pharmacodynamics model is considered in which we consider Cmax as the desired concentration in the biophase and Vd (distribution volume) as the volume of the vitreous humor in which the medicament is going to be injected, the necessary dose to be administered of the medicament is thus obtained:

$$Cmax = D/Vd, \text{ clearing } D = Cmax \times Vd$$

$$D = 0.25 \ \mu g/\mu l \times 57 \ \mu l = 14.3 \ \mu g$$

Therefore in order to obtain a therapeutic concentration of acetylsalicylate in the vitreous of the rat, we must inject 15 micrograms of medicament.

However, we cannot forget that the eye does not follow a strict monocompartimental model since following the injection of the medicament, there is certain spreading of the same to the lens and aqueous humor, such that it is likely that we will encounter a greater distribution volume to that initially considered. If we consider that the lens of the rat has a greater volume than that of the vitreous, it is very likely that the actual distribution volume is closer to 100 microliters, in which case the dose to be used in order to be maintained in a therapeutic range would be 25 micrograms of salicylate which was prepared in 5 $\mu l$ of solution at a concentration of 5 $\mu l/\mu g$ (or 5 mg/ml).

In order to prevent the composition from degrading, we decided to inject the solution immediately after preparing it. The solution used in the experimental test can be prepared as follows:

Pharmaceutical form: sterile solution.

Formula:

| Lysine acetylsalicylate | 900 mg |
| Water for injection | 5 ml |
| Physiological saline solution | 9.5 ml |

Material: 1.5 ml syringes, needles, 0.22 micron filter, sterile bottle.

Preparation: laminar flow hood,

1. Load 5 ml of sterile water for injection and inject it into the vial which contains 900 ml of lysine acetylsalicylate. Stir gently until completely dissolved, thus obtaining a concentration of acetylsalicylic acid of 100 mg/ml of composition = lysine acetylsalicylate 180 mg/ml of composition.

2. Load 0.5 ml (90 mg of lysine acetylsalicylate) of the solution obtained and introduce it into a sterile vial, add 17.5 ml of physiological saline solution, thus obtaining a concentration of 5 mg/ml of composition (5 $\mu g/\mu l$) with a pH of 6.

3. The final preparation will be filtered (0.22 micron filter) prior to packaging it in the Hamilton syringe which will be loaded with a volume of 5 $\mu l$ and covered with a sterile cover.

4. Package and label. The label will indicate the route of administration, the active ingredient, the final concentration, the preparation date, the way of storage and the expiration date.

Packaging: Syringe of suitable volume.

4. Immunohistochemical study

Laminar sections of the retina of all the animals were carried out, being exposed to the action of 4% formaldehyde preservatives. For the marking of the endothelial cells, an anti-ICAM1 monoclonal antibody was used, we also used anti-CD18 monoclonal antibodies for the staining of the leucocytes. After fixing the different sections of stained tissue, the positively stained molecules and the amount of blood vessels present were counted under an optical microscope (Leica® microsystem) with 10X magnification.

For the purposes of assessing significant differences between the different regions of the retina, in all the tissues, the cell and vessel counts corresponding to the central retina (between optical nerve and macular area) and those corresponding to the peripheral retina (outside of the vascular arches) were carried out separately. These counts were specifically carried out on two layers of the retina: the external plexiform layer and the ganglion cell layer. Both retinal layers are those which have demonstrated greater involvement in the inflammatory etiology and vascular affectation in diabetic retinopathy (see Figure 1, Figure 2 and Figure 3).

5. Statistical study

The U Mann-Whitney contrast test was used for independent samples. To contrast the correlation between the variables, the Spearman test was used.

Results

For the analysis, 108 tissue samples were included, 56 corresponding to the specimens treated with the intravitreal medicament and 52 corresponding to the untreated animals and those in which diabetic retinopathy developed naturally.

Central retina

In the treated animals, there are significantly greater counts of CD18, ICAM-1 and vascular density in the external plexiform layer of the central retina, the same occurs in the ganglion layer except in the case of CD18 where there is a greater count in the control animals.

Peripheral retina

A significant increase also occurs in the count of all the variables in the treated group with respect to the control group in the external plexiform layer of the peripheral retina. There is a greater count of ICAM-1 in the treated group and also a greater density of vessels in the ganglion layer. Similar to what occurs in the central retina, the CD18 count in the ganglion layer is not significant in difference from one group to another (see results in Table 1).

Table 1

| VARIABLE | CASE | CONTROL | p value* |
|---|---|---|---|
| **Central retina** | | | |
| **External plexiform** | | | |
| CD18 | 16.5 (14.0-18.0) | 12.0 (9.3-15.0) | <0.001 |
| ICAM1 | 15.0 (14.0-17.5) | 4.0 (2.0-5.0) | <0.001 |
| VESSELS | 9.0 (7.0-11.0) | 3.0 (2.0-4.0) | <0.001 |
| **Ganglion** | | | |
| CD18 | 5.0 (3.0-6.0) | 7.0 (4.3-8.0) | <0.001 |
| ICAM1 | 4.0 (3.0-5.0) | 2.0 (1.3-4.0) | <0.001 |
| VESSELS | 5.0 (2.8-6.0) | 2.0 (1.0-3.0) | <0.001 |
| **Peripheral retina** | | | |
| **External plexiform** | | | |
| CD18 | 8.0 (6.0-9.0) | 4.0 (3.0-5.0) | <0.001 |
| ICAM1 | 9.0 (7.0-11.0) | 3.0 (2.0-4.8) | <0.001 |
| VESSELS | 6.0 (5.0-7.0) | 1.0 (1.0-2.0) | <0.001 |
| **Ganglion** | | | |
| CD18 | 3.0 (2.0-4.0) | 3.0 (2.0-4.0) | 0.972 |
| ICAM1 | 2.0 (1.3-3.0) | 1.0 (1.0-2.0) | <0.001 |
| VESSELS | 3.0 (1.8-4.0) | 1.0 (1.0-2.0) | <0.001 |
| * U Mann-Whitney test for independent samples | | | |

The possible relation between CD18 and vessels has been studied. In the controls, there is no relation between both variables, but there is a relation in the treated animals.

Figure 4, Figure 5, Figure 6 and Figure 7 show the correlation and the result of the Spearman test.

The relation between ICAM1 and vessels has also been studied. There is only a relation in cases for the ganglion layers (see Figure 8 and Figure 9).

Given that it only seems that there may be a relation between CD18 and vessels for the plexiform and ganglion layers of both the central retina and peripheral retina for the treated animals, a model was needed to attempt to explain said relation in these assumptions. The model was created by linear regression:

$$\text{Central retina: CD18} = 0.950 + 0.800 * \text{Vessels (p= 0.002)}$$

$$\text{Peripheral retina: CD18} = 1.066 + 0.536 * \text{Vessels (p<0.001)}$$

*Discussion*

ICAM-1

One of the main problems with the natural development of diabetic retinopathy is the death and disappearance of the cells of vascular endothelium which leads to a change in the integrity of the vessels and to an increase in their permeability. The transmembrane molecule ICAM-1 is expressed almost exclusively in the cell wall of vascular endothelium, being the connection and communication element between the endothelial cell and other cells coming from the blood flow and which contain specific bonds for this protein. The immunohistochemical staining of ICAM-1 which has been carried out reveals that there is a significantly higher count of endothelial cells in the treated animals with respect to those who received no dose of intravitreal salicylate; this would demonstrate the treatment's beneficial effect on the survival of the endothelial cells with respect to the control group. This data is maintained significantly in all the sections of the retina studied both in those corresponding to the macular area and the more peripheral retina.

A large number of endothelial cells would imply greater integrity of the barrier function of the endothelium and therefore greater control of vascular permeability, this invokes a direct beneficial effect of the medicament on the treatment of macular edema in diabetic retinopathy.

Vascular density

The obliteration of the vascular wall is another one of the most damaging phenomena of all those which occur in diabetic retinopathy. It leads to direct obstruction of the arrival of oxygen and nutrients to the retinal microcirculation and therefore to the cells of the retina. This leads to an ischemia of the tissue and cell necrosis which affects both photoreceptors and Müller cells amongst others. In addition, ischemia triggers a cascade of cell events among which include the synthesis and release of a larger amount of vascular endothelium growth factor (VEGF) whose main function is the proliferation of new vessels which can supply those which are not functioning anymore. One problem is that these neovessels which grow on the inner surface of the retina not only do not fulfil their hypothetical function, but they also cause serious complications in the retina, such as constant bleeding which inundates the vitreous humor and the arrival of glial cells to its surface, thus forming large "bridges" of fibrovascular proliferation which can cause the retina to detach due to the traction exerted on it. Another effect of a greater production of VEGF is the increase of vascular permeability which has been associated with the same and its involvement in the formation of diabetic macular edema.

The number of functional vessels and those with cell content in the lumen thereof is significantly greater in each tissue section and in each retina layer included in the study, in the group of animals treated with salicylate. This demonstrates a vessel-protector action of the medicament which could have a significant repercussion on the natural development of diabetic retinopathy, preventing not only the appearance of retinal ischemia both peripheral and central or macular ischemia, but also with implications on the development of macular edema.

CD18

Different studies have shown a significant participation of the leucocytes in the pathogeny of diabetic retinopathy. The destruction of the endothelial cells, the appearance of ischemic retinal areas, the presence of an increase of permeability in the vessels and consequently the appearance of edema and exudates promote the synthesis and release of cytokines which act as chemotactic factors referring to the cells directly involved in the inflammation: the leucocytes.

Acetylsalicylate does not have a direct inhibitory effect on the leucocytes, that is, it neither destroys them nor modulates their arrival to the tissues. But it can interfere in other processes such as blocking enzymatic cascades which promote the synthesis and release of interleukins which act as chemotactic factors, in addition to reducing the vascular and endothelial damage which occurs in this pathology which would also reduce the pro-inflammatory environment present in the tissue.

CD18 is a protein present on the surface of the leucocytes and whose main function is to serve as an anchor to the vascular endothelium prior to migrating from here to the interior of the tissues. In our study, we used a selective antibody against CD18 with the aim of assessing whether the salicylate produces some change in the overall number of leucocytes that infiltrate the retina.

The Spearman correlation test found a significant and directly proportional relation between the vascular density and the count of CD18. A mathematical relation can be established between these two factors with the following formula:

Central retina: CD18 = 0.950 + 0.800*Vessels (p= 0.002)

Peripheral retina: CD18 = 1.066 + 0.536* Vessels (p<0.001)

If we address the mathematical model of correlation, for each vessel present in each tissue section of the plexiform layer of the central retina, there should be 2 leucocytes (1.75); however, this is only fulfilled in the control animals where we observed a vessel count range of between 1-3 and a CD18 leucocytes count range of 4-8. In the treated animals, the range of vessels per tissue section ranges from 3-6 with an average of 5, while the CD18 count ranges from 3-6 with an average of 5. Therefore, according to the mathematical correlation, it is expected that in the central retina of the treated animals there would be at least 10 leucocytes on average per field (taking into account that the expected relation between both variables is 2 leucocytes / 1 vessel). That is, in absolute terms there are more leucocytes per field in the treated group, but in terms correlating to the number of vessels per field, there are half the leucocytes which would be expected if the treated group had developed like the control group. This indicates a real inhibition of the number of leucocytes in the group treated with salicylate.

With all the previous data, it seems clear that there is a main effect of the medicament on the maintenance of the endothelial and vascular integrity which can have significant implications on the natural development and prognosis of the disease relating to both macular edema and retinal ischemia. In addition, the correlation studies reveal that there is a significant reduction in the number of CD18 leucocytes with respect to what was expected given the abundance and well-preserved vascular network in the treated animals.

According to the Wisconsin study, the prevalence at 20 years of macular edema in type 1 diabetics is 29% and in type 2 diabetics is 28%, this percentage of patients being the one susceptible to pharmacological treatment with ranibizumab.

Therefore, while ranibizumab is only indicated for treatment of macular edema secondary to diabetic retinopathy, salicylate has demonstrated a preservation effect in the intraretinal vascular network which suggests that its application would not only allow the macular edema to be controlled and improved, but also prevent ischemia of the more peripheral retina, having a therapeutic value for all patients with diabetic retinopathy, whether they have macular edema, vascular alterations or retinal ischemia.

In addition, the tissues were carefully analyzed in search of signs of medicament toxicity such as de-structuring of the retinal layers, absence of photoreceptor cells or histological alterations of the optical nerve. No tissues were found to have signs that showed alterations suggesting toxicity.

EXAMPLE 2

Taking into account that the volume of the vitreous humor in humans is 4 ml (J Sebag. Vitreous anatomy, aging, and anomalous posterior vitreous detachment. Encyclopedia of the Eye. 2010;307-315.) and following the methodology and the experimental evidence described in example 1, a solution of lysine acetylsalicylate can be prepared for achieving intraocular concentrations in the range of 15 to 30 mg/dl, more preferably aiming for a concentration of 25 mg/dl (0.25 mg/ml).

$$D = Cmax \times Vd$$

$$D = [0.15\text{-}0.30] \text{ mg/ml} \times 4 \text{ ml}$$

To prepare the solution of lysine acetylsalicylate in order to be injected into the human vitreous, a dose of the active ingredient ranges from 0.6 mg and 1.2 mg can be used, a dose of 0.9 mg being preferred. The concentration of

**EP 3 305 300 B1**

the resulting solution should preferably ranges from 12 mg/ml and 24 mg/ml and more preferably 18 mg/ml with a minimal injection volume of 0.05 ml (How to give intravitreal injections. ME Wilson, AW Scott. AAO Eyenet. 2013) and a pH of 6.

The solution described can be prepared as follow:

Pharmaceutical form: sterile solution.

Formula:

| | |
|---|---|
| Lysine acetylsalicylate | 900 mg |
| Water for injection | 5 ml |
| Physiological saline solution | 6 ml |

Material: 1.5 ml syringes, needles, 0.22 micron filter, sterile bottle.

Preparation: laminar flow hood,

1. Load 5 ml of sterile water for injection and inject it into the vial which contains 900 ml of lysine acetylsalicylate. Stir gently until completely dissolved, thus obtaining a concentration of acetylsalicylic acid of 100 mg/ml of composition = lysine acetylsalicylate 180 mg/ml of composition

2. For a solution of 12 mg/ml: load 0.4 ml (72 mg of lysine acetylsalicylate) of the solution obtained and introduce it into a sterile vial, add 5.6 ml of physiological saline solution with a resulting pH of 6.

3. For a solution of 24 mg/ml: load 0.4 ml (72 mg of lysine acetylsalicylate) of the solution obtained and introduce it into a sterile vial, add 2.6 ml of physiological saline solution with a resulting pH of 6.

4. For a more preferred solution of 18 mg/ml: load 0.5 ml (90 mg of lysine acetylsalicylate) of the solution obtained and introduce it into a sterile vial, add 4.5 ml of physiological saline solution with a resulting pH of 6.

5. The final preparation will be filtered (0.22 micron filter) prior to packaging it in the 1 ml syringe which will be loaded with a volume of 0.05 ml and covered with a sterile cover.

6. Package and label. The label will indicate the route of administration, the active ingredient, the final concentration, the preparation date, the way of storage and the expiration date.

Packaging: Syringe of suitable volume.

**Claims**

1.  A composition comprising lysine acetylsalicylate for use in the treatment of a disease of the retina, wherein the composition is administered intravitreally and the disease of the retina is diabetic retinopathy.

2.  The composition for use according to claim 1, wherein the concentration of lysine acetylsalicylate is between 12 mg/ml of composition and 24 mg/ml of composition.

3.  The composition for use according to any of claims 1 or 2, wherein the concentration of lysine acetylsalicylate is 12 mg/ml of composition, 18 mg of composition or 24 mg/ml of composition.

4.  The composition for use according to any of claims 1 to 3, wherein the pH of the composition is between 6 and 6.2.

5.  The composition for use according to claim 4, wherein the pH of the composition is 6.

6.  The composition for use according to any of claims 1 to 5, further comprising physiological saline solution.

**Patentansprüche**

1.  Zusammensetzung, die Lysinacetylsalicylat umfasst, zur Verwendung bei der Behandlung einer Erkrankung der Netzhaut, wobei die Zusammensetzung intravitreal verabreicht wird und die Erkrankung der Netzhaut diabetische Retinopathie ist.

2. Zusammensetzung zur Verwendung nach Anspruch 1, wobei die Konzentration von Lysinacetylsalicylat zwischen 12 mg/ml der Zusammensetzung und 24 mg/ml der Zusammensetzung liegt.

3. Zusammensetzung zur Verwendung nach einem der Ansprüche 1 oder 2, wobei die Konzentration von Lysinacetylsalicylat 12 mg/ml der Zusammensetzung, 18 mg der Zusammensetzung oder 24 mg/ml der Zusammensetzung beträgt.

4. Zusammensetzung zur Verwendung nach einem der Ansprüche 1 bis 3, wobei der pH-Wert der Zusammensetzung zwischen 6 und 6,2 liegt.

5. Zusammensetzung zur Verwendung nach Anspruch 4, wobei der pH-Wert der Zusammensetzung 6 beträgt.

6. Zusammensetzung zur Verwendung nach einem der Ansprüche 1 bis 5, ferner umfassend physiologische Kochsalzlösung.


**Revendications**

1. Composition comprenant de l'acétylsalicylate de lysine destinée à être utilisés pour le traitement d'une maladie de la rétine, dans laquelle la composition est administrée par voie intravitréene et la maladie de la rétine est la rétinopathie diabétique.

2. Composition destinée à être utilisée selon la revendication 1, dans laquelle la concentration d'acétylsalicylate de lysine est comprise entre 12 mg/ml de la composition et 24 mg/ml de la composition.

3. Composition destinée à être utilisée selon l'une quelconque des revendications 1 ou 2, dans laquelle la concentration d'acétylsalicylate de lysine est 12 mg/ml de la composition, 18 mg de la composition ou 24 mg/ml de la composition.

4. Composition destinée à être utilisée selon l'une quelconque des revendications 1 à 3, dans laquelle le pH de la composition est compris entre 6 et 6,2.

5. Composition destinée à être utilisée selon la revendication 4, dans laquelle le pH de la composition est de 6.

6. Composition destinée à être utilisée selon l'une quelconque des revendications 1 à 5, comprenant, en outre une solution saline physiologique.

FIG. 1

FIG. 2

FIG. 3

FIG. 4

FIG. 5

FIG. 6

FIG- 7

FIG. 8

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **L ZHENG ; SJ HOWELL ; DA HATALA ; K HUANG ; T S KERN.** Salicylate-based anti-inflammatory medicaments inhibit the early lesion of diabetic retinopathy. *Diabetes,* 2007, vol. 56, 337-335
- **W SUN ; C GERHARDINGER ; Z DAGHER ; T HOEHN ; M LORENZI.** Aspirin at low-intermediate concentrations protects retinal vessels in experimental diabetic retinopathy through non-platelet-mediated effects. *Diabetes,* 2005, vol. 54, 3418-3426
- **TS KERN ; RL ENGERMAN.** Pharmacological inhibition of diabetic retinopathy, aminoguanidine and aspirin. *Diabetes,* 2001, vol. 50, 1636-1642
- **KRALINGER M. T et al.** *Graefe's Arch Clin Exp Ophthalmol,* 2001, vol. 239, 208-216

- **ABRAMS GW ; AZEN SP ; MCCUEN BW ; FLYNN H ; LAI MY ; RYAN SJ.** Silicone Study Group: Vitrectomy with silicone oil or long-acting gas in eyes with severe proliferative vitreoretinopathy: Results of additional long-term follow-up (Silicone Study Report #11). *Archives of Ophthalmology,* 1997, vol. 115, 335-344
- **ANANT PAI et al.** Current concepts in intravitreal drug therapy for diabetic retinopathy. *Saudi Journal of Ophthalmology,* 2010, vol. 24 (4), 143-149
- **DAO-YI YU ; STEPHEN J. CRINGLE.** Oxygen Distribution in the Mouse Retina. *Invest Ophthalmol Vis Sci.,* 2006, vol. 47, 1109-1112
- **J SEBAG.** Vitreous anatomy, aging, and anomalous posterior vitreous detachment. *Encyclopedia of the Eye,* 2010, 307-315